**Europäisches Patentamt**

(19) **European Patent Office**

**Office européen des brevets**

(11) Veröffentlichungsnummer: **0 011 168**
A1

(12) ## EUROPÄISCHE PATENTANMELDUNG

(21) Anmeldenummer: 79104174.2

(22) Anmeldetag: 29.10.79

(51) Int. Cl.³: **C 07 G 7/00**
A 61 K 39/12, C 12 N 7/04

(30) Priorität: 04.11.78 DE 2847959

(43) Veröffentlichungstag der Anmeldung:
28.05.80 Patentblatt 80/11

(84) Benannte Vertragsstaaten:
AT BE DE NL

(71) Anmelder: BEHRINGWERKE AKTIENGESELLSCHAFT
Postfach 1140
D-3550 Marburg·Lahn(DE)

(72) Erfinder: Reichert, Edgar, Dr.
Flattich-Strasse 3
D-7128 Lauffen (Neckar)(DE)

(74) Vertreter: Reuter, Johann-Heinrich, Dr. et al,
HOECHST AKTIENGESELLSCHAFT Zentrale
Patentabteilung Postfach 80 03 20
D-6230 Frankfurt'Main 80(DE)

(54) Verfahren zur Isolierung von Virusantigenen mittels Polyäthylenglykol aus Lösungen, die nicht-ionische Detergentien enthalten.

(57) Virusantigene werden mittels Polyäthylenglykol aus nicht-ionischen Detergentien enthaltenden Lösungen isoliert durch Zusetzung eines niederen Alkoholes bis zur Klärung der trüben Lösung und Abtrennung der präzipitierten Antigene.

BAD ORIGINAL

EP 0 011 168 A1

Croydon Printing Company Ltd

BEHRINGWERKE AKTIENGESELLSCHAFT    HOE 78/B 016  Dr.HA/wö

Verfahren zur Isolierung von Virusantigenen mittels Polyäthylenglykol aus Lösungen, die nicht-ionische Detergentien
enthalten

Gegenstand der Patentanmeldung ist ein Verfahren zur Isolierung von Virusantigenen.

Es ist bereits vorgeschlagen worden, Virusantigene aus
detergenzienhaltigen Lösungen mit Polyäthylenglykol auszufällen (Patentanmeldung P 27 50 045.4 - HOE 77/B 025 =
Ma 311).

Enthalten die virusantigenhaltigen Lösungen höhere Konzentrationen von nicht-ionischen Detergentien und werden zur
Fällung höhere Polyäthylenglykol-Konzentrationen verwendet,
so wird der Fällungsansatz viskös und trübe. Seine Weiterbehandlung ist verlustreich und nach der Zentrifugation zur
Sedimentation des gefällten Antigens ist der Überstand in
Abhängigkeit von der Konzentration des Detergenz und des
Polyäthylenglykols mehr oder weniger zähflüssig und kann
nur mit Schwierigkeiten abgegossen werden.

0011168

Es wurde nun überraschend gefunden, daß die Trübung verschwindet und die Lösung dann flüssig wird, wenn man ihr einen niederen Alkohol zusetzt.

Gegenstand der Erfindung ist demgemäß ein Verfahren zur Isolierung von Virusantigenen mittels Polyäthylenglykol aus Lösungen in Gegenwart von nicht-ionischen Detergentien, dadurch gekennzeichnet, daß man zu der detergenzhaltigen Virusantigen-Polyäthylenglykol-Mischung einen niederen Alkohol bis zur Klärung der trüben Lösung zusetzt und dann die präzipierten Antigene abtrennt.

Als Alkohole im Sinne der Erfindung kommen niedere, vorzugsweise aliphatische Alkohole in Frage, die eine verhältnismäßig niedrige Viskosität besitzen. Besonders bevorzugt sind einwertige aliphatische Alkohole mit 1 - 4 C-Atomen wie Methanol, Äthanol, n- und i-Propanol sowie die verschiedenen isomeren Butanole.

Die zuzusetzende Menge an Alkohol richtet sich nach dem beabsichtigten Effekt. Durch einfaches Ausprobieren läßt sich die Menge für jeden denkbaren Fall leicht ermitteln, indem man solange kleine Portionen zusetzt, bis die Trübung sich aufgelöst hat und der gewünschte niedrige Viskositätsgrad erreicht ist. Im allgemeinen werden etwa 5 - 40 % (Vol/Vol), zuweilen auch darüber benötigt. Da die zur Aufhebung der Viskosität und der Trübung des Fällungsansatzes notwendige Alkoholkonzentration von der Konzentration des Detergenz und des Polyäthyleglykols im Fällungsansatz abhängig ist, können einige Richtwerte gegeben werden, die aus der folgenden Tabelle zu ersehen sind.

Tabelle

Abhängigkeit der notwendigen Alkoholmenge in % von Detergenz- und Polyäthylenglykol-Gehalt für Methanol, Äthanol, Propanol und Isopropanol. Die Tabelle gilt für Polyäthylenglykol 6000, Polyäthylnglykol 20000 und Polyäthylenglykol 40000 sowie für Triton N-101, Triton X-100 und Arkopal N-110. Die
Prozentangaben beziehen sich auf Volumina.

| Detergenz in % | Polyäthylengkylol in % | | | | | | | |
|---|---|---|---|---|---|---|---|---|
| | 6 | 8 | 12 | 14 | 16 | 18 | 20 | 22 |
| 1 | - | - | 2 | 6 | 9 | 10 | 12 | 14 |
| 2 | - | - | 5 | 8 | 11 | 14 | 18 | 22 |
| 4 | - | - | 6 | 10 | 12 | 15 | 20 | 26 |
| 8 | - | - | 9 | 11 | 13 | 16 | 22 | 32 |

Die für die Durchführung des erfindungsgemäßen Verfahrens
notwendigen Konzentrationen sind für alle erwähnten Alkohole gleich.

Nach Zentrifugation des klaren Fällungsansatzes läßt sich
der dünnflüssige Überstand leicht vom Virusantigen-Sediment
abgießen. Es wird dadurch die Voraussetzung geschaffen,
Fällungen mit Polyäthylenglykol-Konzentrationen auszuführen, bei denen die Isolierung des wertvollen Virusantigens
wegen der Zähflüssigkeit Schwierigkeiten bereitet und nur
unter erheblichen Substanzverlusten möglich wäre.

Beispiel 1

Zu einer Influenzavirus-Oberflächenantigensuspension mit
16 000 Internationalen Einheiten Hämagglutinin/ml in physiologischer Kochsalzlösung, die durch Behandlung von einer
Influenzavirus-Suspension mit 6 % Triton N-101 entstanden
ist, werden 18 % Polyäthylenglykol 6000 zugesetzt.
Der Ansatz wird über Nacht bei 4°C gerührt. Dabei fallen
die Virusantigene aus und die Mischung wird trüb und viskös.
Dann werden dem Ansatz 16 % Äthanol bei Raumtemperatur und
unter Rühren zugesetzt. Der Fällungsansatz wird dadurch
klar und dünnflüssig. Nach Zentrifugation mit 3000 UpM über
30 Minuten wird der Überstand abgegossen. Das Sediment wird in einer Pufferlösung aufgenommen. Es enthält die Virusantigene.

Beispiel 2

Influenzaviren werden gemäß Beispiel 1 mit 6 % Triton N-101 gespalten, mit 14 % Polyäthylenglykol 6000 gerührt, mit 10 % Methanol geklärt und die Virusantigene durch Zentrifugation isoliert.

Beispiel 3

Influenzaviren werden gemäß Beispiel 1 mit 6 % Triton N-101 gespalten, mit 18 % Polyäthylenglykol 6000 gerührt, mit 16 % Propanol geklärt und die Virusantigene durch Zentrifugation isoliert.

Beispiel 4

Influenzaviren werden gemäß Beispiel 1 mit 4 % Triton N-101 gespalten, mit 20 % Polyäthylenglykol 6000 gerührt, mit 20 % Isopropanol geklärt und die Virusantigene durch Zentrifugation isoliert.

Beispiel 5

Influenzaviren werden gemäß Beispiel 3 mit 8 % Triton N-101 gespalten, mit 18 % Polyäthylenglykol 20000 gerührt, mit 16 % Propanol geklärt und die Virusantigene durch Zentrifugation isoliert.

Beispiel 6

Influenzaviren werden gemäß Beispiel 3 mit 8 % Triton N-101 gespalten, mit 18 % Polyäthylenglykol 40000 gerührt, mit 16 % Propanol geklärt und die Virusantigene durch Zentrifugation isoliert.

0011168

Beispiel 7

Influenzaviren werden gemäß Beispiel 1 mit 6 % Triton X-100 gespalten, mit 18 % Polyäthylenglykol 6000 gerührt, mit 16 % Äthanol geklärt und die Virusantigene durch Zentrifugation isoliert.

Beispiel 8

Influenzaviren werden gemäß Beispiel 1 mit 6 % Arkopal N-110 gespalten, mit 18 % Polyäthylenglykol 6000 gerührt, mit 16 % Äthanol geklärt und die Virusantigene durch Zentrifugation isoliert.

Beispiel 9

Die gleichen Ergebnisse werden erzielt, wenn gemäß Beispiel 1 gearbeitet wird und als Detergenz Triton X-100, Arkopal N-060, Arkopal N-080, Arkopal N-110 oder Genapol S-080 und als Virus ein Influenza-Virus B Hongkong; ein Sendai-Virus, Stamm 52; Tollwutvirus, Stamm Pitman Moore oder Herpes simplex virus I oder II eingesetzt wird.

001116

Patentansprüche:

1. Verfahren zur Isolierung von Virusantigenen mittels Polyäthylenglykol aus Lösungen in Gegenwart von nicht-ionischen Detergentien, dadurch gekennzeichnet, daß man zu der detergenzhaltigen Virusantigen-Polyäthylenglykol-Mischung einen niederen Alkohol bis zur Klärung der trüben Lösung zusetzt und dann die präzipitierten Antigene abtrennt.

2. Verfahren nach Anspruch 1, dadurch gekennzeichnet, daß der Alkohol ein einwertiger aliphatischer Alkohol mit 1 - 4 C-Atomen ist.

3. Verfahren nach Anspruch 1 oder 2, dadurch gekennzeichnet, daß die präzipitierten Antigene durch Zentrifugation abgetrennt werden.

# EUROPÄISCHER RECHERCHENBERICHT

## EINSCHLÄGIGE DOKUMENTE

KLASSIFIKATION DER
ANMELDUNG (Int Cl)

| Kategorie | Kennzeichnung des Dokuments mit Angabe, soweit erforderlich, der maßgeblichen Teile | Betrifft Anspruch |
|---|---|---|
| | CHEMICAL ABSTRACTS, Band 73, Nr. 5, 3. August 1970, Zusammenfassung Nr. 23530u, Seite 203, Columbus, Ohio, US, M.J. CORBEL et al : "Soluble antigens obtained from influenza virus by treatment with nonionic detergent" & J.Hyg. 1970, 68(1),81-96 * Zusammenfassung * | 1-3 |
| | -- | |
| | DE - A - 2 140 739 (SOUTH AFRICAN INVENTIONS DEVELOPMENT CORP.) * Ansprüche 1,20,22; Seite 11, Absatz 2 * | 1-3 |
| | -- | |
| | FR - A - 1 350 895 (SOUTH AFRICAN INVENTIONS DEVELOPMENT CORP.) * Ansprüche 1-12; Seite 3, linke Spalte, Zeilen 46-52; Seite 5, linke Spalte, Zeilen 12-18 * | 1-3 |
| | -- | |
| DP | EP - A - 0 001 838 (BEHRINGWERKE AG) * Ansprüche * & DE - A - 2 750 045 | 1-3 |
| | ---- | |

C 07 G 7/00
A 61 K 39/12
C 12 N 7/00

RECHERCHIERTE
SACHGEBIETE (Int Cl)

C 12 N 7/00
A 61 K 39/12
C 07 G 7/00

KATEGORIE DER
GENANNTEN DOKUMENTE

X: von besonderer Bedeutung
A: technologischer Hintergrund
O: nichtschriftliche Offenbarung
P: Zwischenliteratur
T: der Erfindung zugrunde
liegende Theorien oder
Grundsätze
E: kollidierende Anmeldung
D: in der Anmeldung angeführtes
Dokument
L: aus andern Gründen
angeführtes Dokument
&: Mitglied der gleichen Patentfamilie, übereinstimmendes
Dokument

Der vorliegende Recherchenbericht wurde für alle Patentansprüche erstellt

| Recherchenort | Abschlußdatum der Recherche | Prüfer |
|---|---|---|
| Den Haag | 18-02-1980 | RYCKEBOSCH |

EPA form 1503.1  06.78

BAD ORIGINAL